# EUROPÄISCHE PATENTSCHRIFT

(11) **EP 3 372 168 B1**
(45) Veröffentlichungstag und Bekanntmachung des Hinweises auf die Patenterteilung: **27.11.2019**
(21) Anmeldenummer: 17193206.4
(22) Anmeldetag: 26.09.2017
(51) Int. Cl.: A61B 6/04, A61B 6/00, A61B 8/08, A61B 8/00

(54) **RÖNTGEN-UNTERSUCHUNGSGERÄT**
X-RAY EXAMINATION DEVICE
APPAREIL D'EXAMEN RADIOGRAPHIQUE

(43) Veröffentlichungstag der Anmeldung: 12.09.2018
(73) Patentinhaber: Siemens Healthcare GmbH, 91052 Erlangen (DE)
(72) Erfinder: Dederichs, David, 91099 Poxdorf (DE); Kunze, Riccardo, 91054 Erlangen (DE); Lötzsch, Christoph, 91330 Bammersdorf (DE); Radicke, Marcus, 90587 Veitsbronn (DE)

(56) Entgegenhaltungen:
- DE-A1- 19 901 724
- DE-A1- 19 921 100
- US-A1- 2005 207 528
- US-A1- 2007 280 412
- US-A1- 2016 242 707

## Beschreibung

Die Erfindung betrifft ein Röntgen-Untersuchungsgerät sowie ein Verfahren zur Positionierung einer Brust in einem Röntgen-Untersuchungsgerät.

Die frühzeitige Erkennung von Brustkrebs ist eine enorme Herausforderung für alle zurzeit existierenden medizinischen bildgebenden Verfahren.

Die Röntgenmammographie und die Röntgentomosyntheseuntersuchung sind die beiden Standardverfahren in der Brustkrebsdiagnostik. Bei beiden Verfahren wird üblicherweise die Patientin instehender Lage positioniert und die Brust in zwei verschiedenen Richtungen komprimiert (Cranio Caudal und Medio Lateral bzw. Medio Lateral Oblique). Die Kompression der Brust ist vor allem für die Mammographie von besonderer Bedeutung, da hierdurch die Dicke des sich überlagernden Brustgewebes reduziert wird und somit eine verbesserte Diagnostik erreicht werden kann.

Die Kompression der Brust erfolgt dabei typischerweise derart, dass die Brust auf dem Röntgendetektor positioniert wird und eine Kompressionsplatte mit bis zu 200N von oben auf die Brust gedrückt wird. Ein großer Nachteil dieser Kompressionsart ist der häufig von den Patientinnen erfahrene Schmerz.

Ein erster Aspekt der vorliegenden Erfindung bezieht sich daher auf das Verringern oder Vermeiden von Schmerzen bei der Kompression bzw. Fixierung der Brust während Röntgenuntersuchungen.

Es herrscht weitgehende Übereinkunft im gesamten medizinischen Forschungsbereich, dass nur durch die geschickte Kombination von verschiedenen Bildgebungsverfahren eine Diagnose mit gleichzeitig hoher Sensitivität und Spezifität erhalten werden kann. Für eine Diagnose mittels bildgebenden Verfahren werden daher neben Röntgenbilder häufig auch Ultraschallbilder herangezogen.

In der Regel wird hierfür zunächst eine Röntgenuntersuchung, z.B. eine Tomosynthese, durchgeführt. Bei der Tomosynthese werden 2D-Röntgenbilder einer Brust unter verschiedenen Aufnahmewinkeln, beispielsweise während einer kreisbogenförmigen Trajektorie der Röntgenstrahlquelle um das Objekt, aufgenommen und zu einem 3D-Datensatz verrechnet. Ausgehend von diesem 3D-Datensatz werden dann Schichtaufnahmen oder Röntgenbilder mit einem beliebigen Schnittansatz durch den 3D-Datensatz erstellt.

Um eine Gewebeveränderung für eine Diagnose besser beurteilen zu können, werden dann gegebenenfalls zusätzlich Ultraschallbilder angefertigt.

Üblicherweise steht oder sitzt die Patientin bei einer Röntgen-Mammographieuntersuchung unmittelbar vor dem Röntgenuntersuchungsgerät und die Brust wird, wie oben beschrieben, zwischen zwei Platten komprimiert, während die Patientin bei einer Ultraschalluntersuchung (Sonographie) auf einer Patientenliege liegt und die Brust durch den Ultraschallkopf an den Brustkorb gedrückt wird. Die Patientin muss daher zwischen der Röntgen- und der Ultraschall-Untersuchung neu positioniert werden. Zusätzlich nachteilig wirkt sich aus, dass die Brust während der Röntgen- und der Ultraschall-Untersuchung unterschiedlich stark sowie in unterschiedlichen Richtungen komprimiert wird. Weiter ist von Nachteil, dass die Röntgen- und die Ultraschall-Bilder aus unterschiedlichen Betrachtungsrichtungen aufgenommen werden und es daher schwierig ist, die so erhaltenen Bilder möglichst genau zu überlagern bzw. zueinander in Verbindung zu setzen.

Es sind Prototypen kombinierter Röntgen-/Ultraschall-Untersuchungsgeräte bekannt. So sind beispielsweise Untersuchungsgeräte bekannt, bei denen eine Kompressionsanordnung eine untere Kompressionseinheit in Form einer den Röntgendetektor aufweisenden Lagerplatte und eine obere Kompressionseinheit in Form einer Kompressionsplatte, beispielsweise eine Plexiglasplatte, umfasst. Derartige Geräte sehen eine Ultraschalluntersuchung an einer Brust vor, die zwischen der oberen Kompressionsplatte und der unteren Lagerplatte des Untersuchungsgerätes komprimiert ist. Dabei wird der für die Sonographie benötigte Ultraschallkopf in einer Abtastbewegung entlang der Tomosynthese-Scanrichtung über die Oberseite der Kompressionsplatte geführt. Aufgrund der im wesentlichen unveränderten Kompression der Brust während beider bildgebender Verfahren sowie aufgrund der definierten und daher zueinander in Beziehung setzbaren Betrachtungsrichtungen ermöglichen die erhaltenen Bilder eine verbesserte Diagnose.

Da die abgestrahlten Ultraschallsignale durch die Kompressionsplatte hindurch in die zu untersuchende Brust eingekoppelt werden müssen, wurde vorgeschlagen, anstelle einer starren Kompressionsplatte eine Kompressionsmulde mit einer flexiblen Kompressionsfläche zu verwenden, wobei diese flexible Kompressionsfläche beispielsweise mit einem als Gaze bezeichneten Gewebe gebildet wird. Die Brust wird durch diese Ausgestaltung der Kompressionsmulde während einer Kompression leicht konvex geformt. Ein Vorteil dieser Art der Kompression ist eine homogenere Verteilung der Kompressionskraft auf das zu untersuchende Objekt sowie die Möglichkeit einer Ultraschalluntersuchung unmittelbar im Anschluss an eine Röntgenuntersuchung ohne die technischen Probleme, die sich aus einer Einkopplung des Ultraschalls durch eine Kompressionsplatte hindurch ergeben würden.

Das Hauptproblem bei Hybridsystemen, die sowohl eine Röntgenals auch eine Ultraschalluntersuchung in einer einzigen Brustpositionierung bewerkstelligen, liegt in der Ankopplung der Brust an den Ultraschallkopf und somit einer guten Abdeckung der Brustfläche während der Ultraschallaufnahme. Typischerweise werden Bereiche der Brust, die nicht beidseitig von den Kompressionsplatten beaufschlagt werden, also insbesondere der brustwandnahen Bereich nahe der Brustwarze, oder mit anderen Worten die Brustvorderseite, trotz einer Komprimierung gar nicht oder nur ungenügend an den Ultraschallkopf angekoppelt, so dass diese Bereiche nicht bildgebend dargestellt werden können. Während bei der Röntgenaufnahme Bilder der gesamten auf dem Röntgendetektor angeordneten Brust aufgenommen werden können, fehlt bei der entsprechenden Ultraschallaufnahme bei identischer Brustpositionierung oftmals der vordere Bereich der Brust, der bis zu 25 Prozent der korrespondierenden Bildfläche der Röntgenaufnahme ausmachen kann. Dies führt dazu, dass die bisher bekannten Hybridsysteme für eine Screening- oder Diagnostiknutzung nur eingeschränkt verwendbar sind.

Ein zweiter Aspekt der vorliegenden Erfindung bezieht sich daher auf eine verbesserte Ultraschallankopplung bei einer kombinierten Röntgen-/Ultraschall-Untersuchung.

Aus DE 199 01 724 A1 ist eine Ankoppeleinheit für Brustuntersuchungen in einem Röntgenmammographiegerät mit gleichzeitiger Mammadiagnostik mit Hilfe von Ultraschall bekannt, wobei an der Kompressionsplatte und/oder an der Lagerungsplatte Koppelkissen befestigt sin, die über Pumpen mit Wasser befüllbar sind und die durch seitliche Abstützungen beim Befüllen sich so verformen, dass sie die Brust allseitig umschließen. Die Kompression der Brust erfolgt dabei ausschließlich über die Platten.

Es ist eine Aufgabe der vorliegenden Erfindung, Röntgenuntersuchungen der Brust zu optimieren. Diese Aufgabe wird durch ein Röntgen-Untersuchungsgerät nach Anspruch 1 bzw. durch ein Verfahren nach Anspruch 5 gelöst. Vorteilhafte Ausführungen der Erfindung sind in den Unteransprüchen angegeben.

Die im Folgenden im Zusammenhang mit dem Röntgen-Untersuchungsgerät erläuterten Vorteile und Ausgestaltungen gelten sinngemäß auch für das erfindungsgemäße Verfahren und umgekehrt.

Ein erfindungsgemäßes Röntgen-Untersuchungsgerät ist als als kombiniertes Röntgen-/Ultraschall-Untersuchungsgerät ausgeführt und umfasst einen Röntgenstrahler und einen Röntgendetektor zur Aufnahme wenigstens eines Röntgenbildes einer Brust und eine Ultraschalleinheit zur Aufnahme wenigstens eines Ultraschallbildes der Brust sowie eine Kompressionseinheit zur Kompression und Fixierung der Brust, wobei die Kompressionseinheit ein für Röntgenstrahlen und für Ultraschall durchlässiges Kompressionselement aufweist, und wobei die Ultraschalleinheit entlang des Kompressionselements über die Brust führbar ist, und ist gekennzeichnet durch ein volumen- und/oder formvariables Positionierelement zur Beaufschlagung der Brust derart, dass das Positionierelement die Brust verformt und sich die Brust an das Kompressionselement anlegt, wobei das Positionierelement als ein befüllbares und entleerbares Kissen ausgeführt ist, das mindestens eine Kammer aufweist und wobei die Volumen- und/oder Formvariabilität des Positionierelements durch ein Befüllen mindestens einer Kammer des Positionierelements mit einem Füllmedium und/oder durch ein Entleeren mindestens einer Kammer des Positionierelements bewirkbar ist.

Ein erfindungsgemäßes Verfahren zur Positionierung einer Brust in einem als kombiniertes Röntgen-/Ultraschall-Untersuchungsgerät ausgeführten Röntgen-Untersuchungsgerät mit einem Röntgenstrahler und einem Röntgendetektor zur Aufnahme wenigstens eines Röntgenbildes einer Brust sowie mit einer Ultraschalleinheit zur Aufnahme wenigstens eines Ultraschallbildes der Brust und mit einer Kompressionseinheit zur Kompression und Fixierung der Brust, wobei die Kompressionseinheit ein für Röntgenstrahlen und für Ultraschall durchlässiges Kompressionselement aufweist und wobei die Ultraschalleinheit entlang des Kompressionselements über die Brust führbar ist, ist dadurch gekennzeichnet, dass eine Positionierung der Brust unter Verwendung eines zur Beaufschlagung der Brust ausgebildeten, volumen- und/oder formvariablen Positionierelements erfolgt derart, dass das Positionierelement die Brust verformt und sich die Brust an das Kompressionselement anlegt, wobei das Positionierelement als ein befüllbares und entleerbares Kissen ausgeführt ist, das mindestens eine Kammer aufweist und wobei die Volumen- und/oder Formvariabilität des Positionierelements durch ein Befüllen mindestens einer Kammer des Positionierelements mit einem Füllmedium und/oder durch ein Entleeren mindestens einer Kammer des Positionierelements bewirkt wird.

Mit der Erfindung werden Schmerzen bei der Kompression bzw. Fixierung der Brust während Röntgenuntersuchungen verringert oder vermieden. Darüber hinaus ermöglicht die Erfindung bei einer kombinierten Röntgen-/Ultraschall-Untersuchung eine verbesserte Ankopplung der Brust an den Ultraschallkopf und damit eine verbesserte Abdeckung für die Ultraschallaufnahme bei identischer Positionierung der Brust.

Eine Kernidee der Erfindung ist es, mit Hilfe des Positionierelements eine definierte Positionierung der Brust für ein medizinisches bildgebendes Verfahren, insbesondere 2D-/3D-Mammographie bzw. Tomosynthese, auch in Kombination mit Ultraschallbildgebung, durchzuführen, indem das Positionierelement als befüllbares und wieder entleerbares Kissen ausgeführt ist, wobei das Befüllen und Entleeren gezielt durchführbar ist derart, dass das Kissen mit einer Kontaktfläche die Brust flächig beaufschlagt und dadurch formt und/oder bewegt. Durch diese Formung und/oder Bewegung der Brust trägt das Positionierelement zu der gewünschten Positionierung der Brust in der Kompressionseinheit bei. Mit anderen Worten bewirkt oder unterstützt das Positionierelement eine Positionierung der Brust in einer gewünschten Bildaufnahmeposition.

Im Ergebnis wird die Brust in der Kompressionseinheit von mehreren Seiten beaufschlagt, nämlich einerseits durch die Kompressionselemente der Kompressionseinheit, also beispielsweise den Röntgendetektor als untere Kompressionsplatte sowie ein bewegliches Kompressionselement, das als obere Kompressionsplatte oder als Gewebe ausgeführt sein kann, somit typischerweise von unten sowie von oben, sowie andererseits durch das form- und/oder volumenveränderbare Positionierelement, das wenigstens an einer Seite der Brust, beispielsweise an der Brustunterseite oder der Brustoberseite, an dieser anliegt, vorzugsweise jedoch mehrseitig an der Brust anliegt, insbesondere auch seitlich, so dass es die Brust nicht nur anheben bzw. herabdrücken sondern auch seitlich beaufschlagen und dadurch in eine gewünschte Form bringen kann. Hierdurch lässt sich die Brust nicht nur auf besonders einfache Weise besser in eine für die Röntgenaufnahme gewünschte Aufnahmeposition bringen. Es kann auch eine besonders homogene bzw. gleichmäßige, vorzugsweise allseitige Kraftverteilung auf die Brust erfolgen, was zu einer verbesserten Fixierung der Brust sowie auch zu verringerten Schmerzen führt. Da anstelle harter Oberflächen nun zumindest anteilig, vorzugweise aber ausschließlich flexible bzw. nachgebende Körper, einschließlich der Gaze, zur Beaufschlagung der Brust mit der erforderlichen Kontaktkraft bzw. dem erforderlichen Druck verwendet werden, wird die erzielte Kompression bzw. Fixierung zudem als besonders "weich" empfunden, was zu einem gesteigerten Patientenkomfort führt. Durch eine geeignete Kraft- bzw. Druckverteilung entsprechend der vorhandenen Brustform bzw. Anatomie kann insbesondere eine unnötig hohe Druckkraft auf brustwandnahe Bereiche der Brust vermieden werden. Bei einer über die gesamte Brust gleichmäßig verteilten Kraftausübung während der Kompression sind diese Bereiche bei den aus dem Stand der Technik bekannten Verfahren besonders hohen Drücken ausgesetzt, was zu Schmerzen bei den Patientinnen führt.

Darüber hinaus wird mit Hilfe des Positionierelements eine für die Ultraschallaufnahme bei einer kombinierten Röntgen-/Ultraschall-Untersuchung vorteilhafte Positionierung der Brust ermöglicht, zusätzlich zu bzw. in Verbindung mit der bereits durch die Kompressionseinheit erfolgten Kompression und Fixierung der Brust. Mit Hilfe des Positionierelements können diejenigen Bereiche der Brust, die bei der herkömmlichen Kompression und Fixierung durch die Kompressionseinheit nicht an der Kompressionsfläche des Kompressionselements anliegen, an diese angelegt werden, so dass auch von diesen Bereichen eine Ultraschallaufnahme angefertigt werden kann, wenn die Ultraschalleinheit entlang des Kompressionselements über die Brust geführt wird. Das Positionierelement dient mit anderen Worten für bestimmte Abschnitte der Brust als zusätzliches Positionierungs- und/oder Fixierungsmittel.

Durch die Kompression der Brust, wie sie für die Durchführung der Röntgenaufnahmen (Mammographie und/oder Tomosynthese) erfolgt, kann mit anderen Worten zugleich eine Fixierung der Brust vorgenommen werden, wie sie für die Durchführung von Ultraschallaufnahmen vorteilhaft ist. Da sich bei Verwendung des erfindungsgemäßen Untersuchungsgerätes weder die Lage noch die Kompression der Brust bei dem Wechsel der bildgebenden Modalitäten verändert, können beide Verfahren bei gleichbleibender Positionierung erfolgen, wodurch Bildinhalte vergleichsweise einfach zueinander in Beziehung gesetzt werden können.

Unter Verwendung des Positionierelements lässt sich auf einfache Weise eine für die Ultraschallankopplung und die gewünschte Brustabdeckung vorteilhafte Positionierung der Brust erreichen. Mit Hilfe des zumindest abschnittsweise an der Brust anliegenden Positionierelement kann die Brust bzw. ein Bereich der Brust angehoben, genauer gesagt von dem Röntgendetektor abgehoben und an die Unterseite des Kompressionselements bewegt werden, bis sie an der Kompressionsfläche anliegt. Herkömmliche Geräte müssen daher für die Umsetzung der Erfindung lediglich durch ein geeignetes Positionierelement sowie einen Befüllungs- und Entleerungsmechanismus ergänzt werden. Die Erfindung lässt sich daher auch an bereits bestehenden Geräten nachrüsten.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Dabei zeigen:
FIG 1 ein kombiniertes Röntgen-/Ultraschall-Untersuchungsgerät von der Seite (Stand der Technik),
FIG 2 ein kombiniertes Röntgen-/Ultraschall-Untersuchungsgerät von der Seite,
FIG 3 ein kombiniertes Röntgen-/Ultraschall-Untersuchungsgerät von vorn.

Sämtliche Figuren zeigen die Erfindung lediglich schematisch und mit ihren wesentlichen Bestandteilen. Gleiche Bezugszeichen entsprechen dabei Elementen gleicher oder vergleichbarer Funktion.

Abgebildet sind schematische Darstellungen von Teilen eines kombinierten Röntgen-/Ultraschall-Untersuchungsgerätes 1, welches sich zur Durchführung von Mammographie- und Tomosyntheseverfahren eignet. Dieses Untersuchungsgerät 1 weist einen entlang einer Trajektorie 2 verfahrbaren Röntgenstrahler 3 mit einem dazugehörigen Röntgendetektor 4 sowie eine Ultraschalleinheit mit einem in der Kompressionseinheit integrierten Ultraschallkopf (Transducer) 5 auf, also eine Ultraschallsonde, welche die Ultraschallwellen sendet und empfängt, diese in elektrische Signale umwandelt und zur Weiterverarbeitung weiterleitet. Alternativ dazu kann die Ultraschalleinheit auch einen Ultraschallsender aufweisen, der mit einem geeignet platzierten Ultraschallempfänger zusammenwirkt (nicht dargestellt).

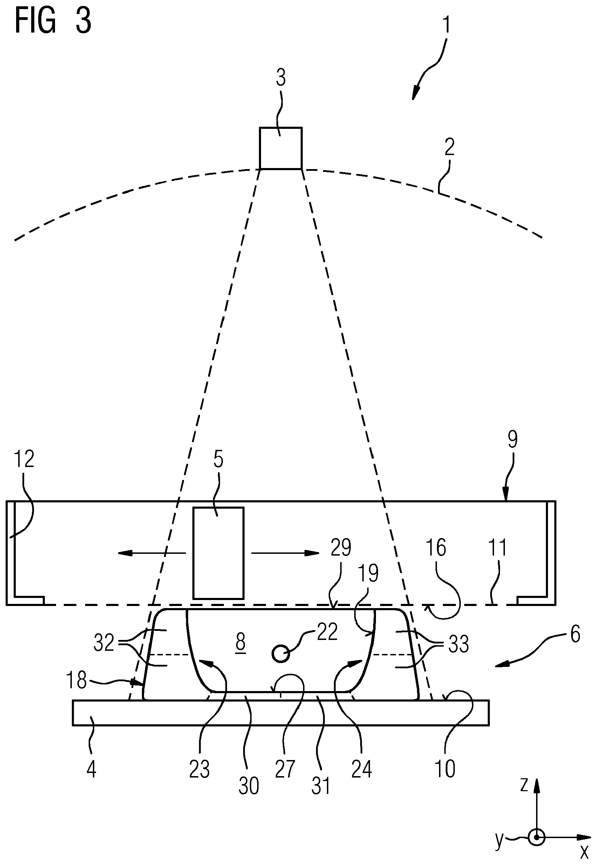

Die Kompressionsanordnung 6 umfasst eine obere Kompressionseinheit und eine untere Kompressionseinheit. Die Kompressionseinheiten sind auf gegenüberliegenden Seiten eines dazwischenliegenden Untersuchungsbereiches 7 positionierbar, wobei der Röntgenstrahler 3 derart positionierbar ist, dass seine Röntgenstrahlen den Untersuchungsbereich 7 durchlaufen und anschließend auf den Detektor 4 treffen. Ein von dem Röntgenstrahler 3 ausgehender, sich in Richtung Detektor 4 ausbreitender Röntgenkegel ist in FIG 3 angedeutet. Während der Röntgenaufnahme ist der Ultraschallkopf 5 außerhalb des Röntgenkegels positioniert.

Im vorliegenden Fall dient der Detektor 4, der als digitaler Flachdetektor ausgeführt sein kann, als untere Kompressionseinheit. Für eine Brustuntersuchung wird die Brust 8 einer Patientin direkt auf dem Detektor 4 positioniert. Mit der oberen Kompressionseinheit, hier in Form einer Kompressionsmulde 9, wird die zu untersuchende Brust 8 zwischen der Kompressionsmulde 9 und der Oberseite 10 des Detektors 4 fixiert und komprimiert. Die Kompressionsmulde 9 ist zum Detektor 4 hin mit einem flexiblen Kompressionselement in Form eines Gewebes 11 ausgebildet, wozu hier Gaze verwendet wird, die in einem Rahmen 12 eingespannt ist. Dieses Gewebe 11 bildet eine für Röntgenstrahlen und Ultraschall durchlässige Kompressionsfläche 16 und ist zur Auflage auf die Brust 8 ausgebildet. Das Gewebe 11 überspannt dabei im Wesentlichen den gesamten Bodenbereich der Kompressionsmulde 9.

Insbesondere kann es vorgesehen sein, das in der Kompressionsmulde 9 gehaltene Gewebe 11 als Einwegartikel auszugestalten. Auf diese Weise muss das Gewebe nach der Verwendung nicht aufwändig von dem Kontaktgel gereinigt werden. Hierzu kann der Rahmen 12 der Kompressionsmulde 9 so ausgeführt sein, dass das Gewebe 11 darin eingespannt wird, insbesondere geeignete Spannelemente aufweisen. Das Gewebe 11 kann aber mit dem Rahmen 12 auch verklebt oder auf andere Weise lösbar daran befestigt sein. Alternativ kann auch der gesamte Rahmen 12 mitsamt dem Gewebe 11 als von der Kompressionsmulde 9 abtrennbares, lediglich einmal verwendbares Bauteil ausgeführt sein.

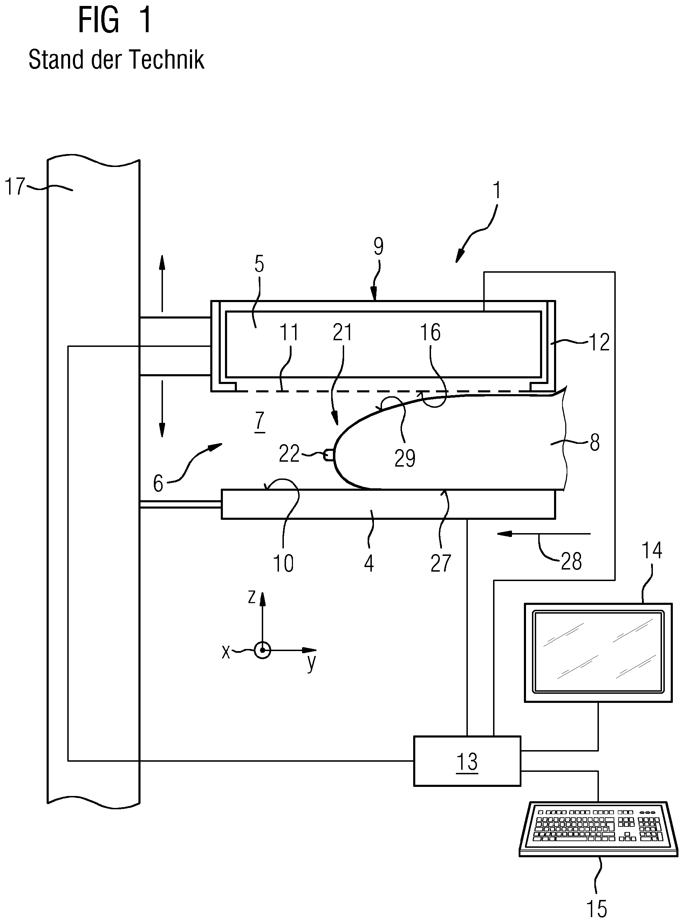

Eine zu dem Untersuchungsgerät 1 gehörende Steuer- und Recheneinheit 13 mit zugehörigem Bildschirm 14 und Eingabeinheit 15 ist in FIG 1 dargestellt. Diese dient zur Steuerung des Untersuchungsgerätes 1, d.h. sowohl zur Steuerung des Röntgenstrahlers 3 und des Detektors 4 als auch zur Steuerung des Ultraschallkopfes 5 und wird darüber hinaus beispielsweise für die Durchführung von Bildverarbeitungsalgorithmen zur 2D- und/oder 3D-Bilderzeugung verwendet. In FIG 1 ist gezeigt, dass bei den im Stand der Technik verwendeten Verfahren der im Bereich der Brustwarze 22 gelegene vordere Bereich 21 der Brust 8 nicht an den Ultraschallkopf 5 angekoppelt ist.

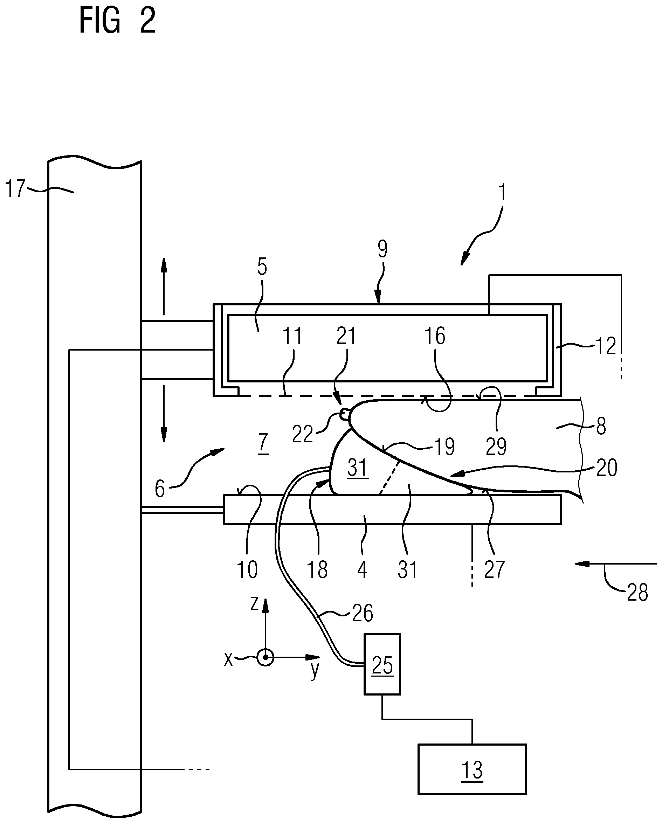

Nachdem die Brust 8 durch die Kompressionsanordnung 6 komprimiert und fixiert ist und nachdem mit Hilfe eines Positionierelements 18 eine zusätzliche Positionierung der Brust 8 erfolgt ist, wie nachfolgend im Zusammenhang mit FIG 2 und 3 genauer erläutert, werden mit dem Röntgenstrahler 3 und dem Detektor 4 erste Bilder von der Brust 8 aufgenommen. Unter Beibehaltung der Komprimierung und Fixierung der Brust 8 können anschließend zweite Bilder mit Hilfe des Ultraschallkopfes 5 aufgenommen werden, indem der Ultraschallkopf 5 entlang der Kompressionsfläche 16 über die Brust 8 geführt wird. Die Komprimierung der Brust 8 wird während der Bildaufnahmen und zwischen der Aufnahme der Bilder beibehalten, ebenso die Position und der Blickwinkel der jeweiligen Aufnahme.

Die einzelnen mit dem Röntgendetektor 4 aufgenommenen 2D-Röntgenbilder können in der Steuer- und Recheneinheit 13 mittels eines Rekonstruktionsverfahrens zu einem 3D-Datensatz verrechnet werden. Röntgenbilder können mit Ultraschallbildern überlagert werden. Eine geeignete Bildverarbeitung dient der Darstellung und Überlagerung oder Fusion der verschiedenen Messergebnisse. Sie erzeugt eine visuelle Darstellung auf dem Bildschirm 14 oder einem anderen Wiedergabegerät. Hierbei können überlagert oder synchron beispielsweise ein Röntgenbild und ein Ultraschallbild dargestellt sein. Es ist dann für einen Arzt besonders einfach möglich, anhand der Röntgen- und Ultraschallbilder mit unveränderter Örtlichkeit das untersuchte Brustgewebe zu analysieren.

Es können verschiedene Röntgenaufnahmen (Mammographie, Tomosynthese) allein oder in Kombination mit Ultraschallaufnahmen durchgeführt werden. Eine Ultraschallaufnahme kann auch unabhängig von einer Röntgenaufnahme erfolgen.

In FIG 2 ist, ebenso wie in FIG 1, schematisch eine Seitenansicht eines Untersuchungsgerätes 1 gezeigt, welches jedoch, anders als in FIG 1, mit einem erfindungsgemäßen Positionierelement 18 ausgestattet ist. An einem vertikalen Trägerelement 17 des Untersuchungsgerätes 1, hier einem Stativ, sind der Detektor 4 sowie die Kompressionsmulde 9 angeordnet. Der Röntgenstrahler 3 kann ebenfalls an dem Trägerelement 17 oder aber an einem separaten Haltearm (nicht dargestellt) des Untersuchungsgerätes 1 angebracht sein. Die Kompressionseinheiten (Detektor 4 und Kompressionsmulde 9) sind derart an dem Trägerelement 17 gelagert, dass die Kompressionsmulde 9 zur Komprimierung einer im Untersuchungsbereich 7 befindlichen Brust 8 herabgesenkt werden kann (angedeutet durch Pfeile). Der Detektor 4 dient vorzugsweise als statische Auflage, kann jedoch ebenfalls höhenverstellbar gelagert sein.

In der Kompressionsmulde 9 ist der auf der Kompressionsfläche 16 aufliegende und dort horizontal als auch vertikal verfahrbare, d.h. über das auf der Brust 8 aufliegende Gewebe 11 geführte Ultraschallkopf 5 platziert. Dieser Ultraschallkopf 5 ist mittels einer Führungs- und Verfahreinheit (nicht dargestellt) horizontal in der durch die Kompressionsmulde 9 definierten Ebene in X- und/oder Y-Richtung verfahrbar (angedeutet durch Pfeile in FIG 3). Ein mit der Führungs- und Verfahreinheit verbundener, zur Bewegung des in der Kompressionsmulde 9 integrierten Ultraschallkopfes 5 vorgesehener motorischer Antrieb ist ebenfalls vorhanden, jedoch nicht dargestellt.

Zur besseren Ankopplung der Ultraschallwellen an die Brust 8 kann auf das jeweilige Kompressionselement, hier das Gewebe 11, mittels einer nicht gezeigten Spendereinheit ein Ultraschallkoppel-Gel (Kontakt-Gel) aufgetragen werden.

Vor der Platzierung der Brust 8 auf dem Röntgendetektor 4 wird dort ein als Positionierelement dienendes, aufblasbares Luftkissen 18 angeordnet, siehe FIG 2. Das Luftkissen 18 kann in einzelne Kammern oder Sektionen unterteilt sein. Vorzugsweise weist das Luftkissen 18 mehrere voneinander getrennte oder voneinander trennbare Kammern 30, 31, ... auf, die wahlweise mit Luft befüllbar oder entleerbar sind, wodurch sich Form und/oder Volumen der Kammern 30, 31, ... und damit Form und/oder Volumen des Luftkissens 18 verändern lässt. Vorzugsweise sind die Kammern 30, 31, ... zu diesem Zweck unabhängig voneinander befüllbar und entleerbar. Anders ausgedrückt lässt sich der Druck innerhalb der Kammern 30, 31, ... kammerindividuell und gezielt verändern. Hierdurch erhält das Luftkissen 18 seine Volumen- und/oder Formvariabilität. Mit anderen Worten ändert sich Form und/oder Volumen des Luftkissens 18 in Abhängigkeit von dem Grad seiner Befüllung. In den FIG 2 und 3 sind aus Gründen der Übersichtlichkeit jeweils nicht alle Kammern 30, 31, ... des Luftkissens 18 dargestellt.

Anstelle von Luft kann auch ein anderes geeignetes Gasgemisch zum Befüllen des Luftkissens 18 verwendet werden. Überhaupt kann, je nach Anwendungsfall und Ausführung des Systems, anstelle eines pneumatischen Befüllungssystems auch ein hydraulisches Befüllungssystem verwendet werden und bei dem Füllmedium kann es sich um ein beliebiges, für diesen Zweck geeignetes Fluid handeln, wobei die nachfolgend im Zusammenhang mit einem Luftkissen 18 erläuterten Vorteile und Eigenschaften entsprechend auch auf andere Ausführungsformen übertragbar sind.

Das Luftkissen 18 stellt an seinen Außenseiten mehrere Anlageflächen 19 für die Brust 8 bereit. Diese dienen, vor, während und/oder im Anschluss an das Herabfahren der oberen Kompressionseinheit 9, zur flächigen Beaufschlagung der Brust 8 zum Zweck ihrer Positionierung in der Kompressionsanordnung 6, d.h. zwischen den beiden Kompressionseinheiten 4, 9, und/oder zum Zweck ihrer Kompression und/oder Fixierung. Anders ausgedrückt beaufschlagen die Anlageflächen 19 des Luftkissens 18 die Brust 8, indem sie sich aufgrund ihrer Befüllung an die Brust 8 anlegen und damit die Brust 8 oder Abschnitte bzw. Teile der Brust 8 in einer bestimmten Position fixieren und/oder indem sie im weiteren Verlauf des Positionierungsvorgangs, insbesondere während bzw. aufgrund einer weiteren Befüllung des Luftkissens 18 und einer Erhöhung des Kammerinnendrucks, die Brust 8 oder Abschnitte bzw. Teile der Brust 8 verformen und/oder in eine bestimmte Zielposition bewegen. Der Zeitpunkt der Befüllung des Luftkissens 18 bzw. das Zeitregime zum Befüllen und/oder Entleeren der einzelnen Kammern 30, 31, ... des Luftkissens 18 wird in Abhängigkeit von der zu positionierenden Brust 8, insbesondere von Größe und Form der Brust 8, und der zu erreichenden Zielpositionierung sowie in Abstimmung mit dem Kompressionsvorgang durch das Herabfahren der Kompressionseinheit 9 definiert.

Unter anderem dient das Luftkissen 18, insbesondere mit seinen unterhalb der Brust 8 angeordneten Kammern 30, 31, ..., zum abschnittsweisen Anheben der darauf liegenden Brust 8 derart, dass sich die Brust 8 in dem angehobenen Brustabschnitt 21 an die oberhalb der Brust 8 angeordnete Kompressionsfläche 16, hier also die Gaze 11, von unten anlegt. Die Brust 8 kann jedoch durch das Luftkissen 18 auch vollständig, also nicht nur abschnittsweise angehoben werden.

Gleichzeitig dient das Luftkissen 18, insbesondere mit seinen seitlich der Brust 8 angeordneten Kammern 32, 33, ..., zum Formen der Brust 8, vorzugsweise derart, dass die Brust 8 vorzugsweise keil- oder kegelfömig in Richtung der Kompressionsfläche 16 bewegt bzw. komprimiert wird, und zwar insbesondere derart, dass die an der Kompressionsfläche 16 anliegende Brustoberfläche größer ist als die auf dem Röntgendetektor 4 aufliegende bzw. in Richtung des Röntgendetektors 4 weisende Brustoberfläche. Auch können die Brustwandbereiche, insbesondere die seitlichen Brustwandbereiche und der vordere Brustwandbereich 21 um die Brustwarze 22 durch eine geeignete Befüllung der Kammern 30, 31, ... des Luftkissens 18 so geformt und ausgebildet werden, dass ihre Lage bzw. ihr Verlauf für die gewünschten Bildaufnahmen optimal sind.

Gegenüber einer herkömmlichen, aus dem Stand der Technik bekannten Kompression mit Hilfe starrer Kompressionsplatten ändert sich bei Anwendung des erfindungsgemäßen Positionierungselements 18 die Brustform der komprimierten Brust 8. Mit anderen Worten weist die erfindungsgemäß komprimierte Brust 8 eine andere Form, insbesondere eine andere Querschnittsform, auf als bei herkömmlichen Verfahren, welche die Brustpositionierung ohne das Positionierungselement 18 durchführen. Darüber hinaus ändert sich unter Umständen auch die Krümmung der Gaze 11, gegen die sich die Brust 8 anlegt.

Die Anzahl und die Anordnung der Kammern 30, 31, ... kann von Luftkissen zu Luftkissen in Abhängigkeit von dem jeweiligen Anwendungsfall variieren. Typischerweise sind untere Kammern 30, 31, ... vorgesehen, die unmittelbar auf dem Röntgendetektor 4 platzierbar sind und auf denen die Brust 8 angeordnet (aufgelegt) wird. Ergänzend sind vorzugsweise seitliche Kammern 32, 33, ... vorgesehen, die seitlich der platzierten Brust 8 angeordnet sind derart, dass sich, in Brustlängsrichtung 28 gesehen, ein im wesentlichen U-förmiger Querschnitt des Luftkissens 18 ergibt, siehe FIG 3. Weiter ergänzend sind vorzugsweise auch vordere Kammern vorgesehen (nicht dargestellt), die sich vor der Brustwarze 22 befinden. Mit Hilfe dieser vorderen Kammern lässt sich die Brust 8 von drei Seiten einfassen.

Es sind vorzugsweise mehrere Kammern 30, 31, ... in Brustlängsrichtung 28 nebeneinander angeordnet, so dass verschiedene Abschnitte der Brust 8 in Brustlängsrichtung 28 unterschiedlich beaufschlagt werden können, also beispielsweise ein hinterer, brustmuskelnaher Abschnitt, ein mittlerer Abschnitt 20 und ein vorderer, brustwarzennaher Abschnitt 21 der Brust 8. Darüber hinaus sind vorzugsweise mehrere Kammern 30, 31, ... quer zu der Brustlängsrichtung 28 nebeneinander angeordnet, so dass verschiedene Abschnitte der Brust 8 quer zu der Brustlängsrichtung 28 unterschiedlich beaufschlagt werden können, also beispielsweise ein in Richtung der Brustwarze 22 gesehen rechter Seitenabschnitt 23 der Brust 8 oder ein in Richtung der Brustwarze 22 gesehen linker Seitenabschnitt 24 der Brust 8.

Durch ein individuelles Aufblasen des Luftkissens 18 ist es mit anderen Worten möglich, mit einer rechts und links bzw. in Querrichtung, also quer zu der Brustlängsrichtung 28, gesehen voneinander verschieden großen Druckkraft an der Brust 8 anzugreifen, so dass beispielsweise die in Brustlängsrichtung 28 gesehen rechte Seite 23 der Brust 8 stärker beaufschlagt wird als die in Brustlängsrichtung 28 gesehen linke Seite 24 der Brust 8, wodurch beispielsweise die rechte Seite 23 der Brust 8 stärker angehoben bzw. stärker an die Kompressionsfläche 16 gedrückt wird als die linke Seite 24 der Brust 8, oder umgekehrt. Hierdurch ist es möglich, das Anheben der Brust 8 bzw. deren Positionierung allgemein und die gewünschte Endposition der Brust 8 an der Kompressionsfläche 16 brustindividuell einzustellen.

Insbesondere die seitlichen Kammern 32, 33, ... und die vorderen Kammern können nicht nur nebeneinander, sondern auch übereinander angeordnet sein. Damit kann beispielsweise ein der Brustunterseite 27 naher Abschnitt der Brust 8 anders beaufschlagt werden als ein der Brustoberseite 29 naher Abschnitt der Brust 8. Im vollständig befüllten Zustand können die Kammern 30, 31, ... eine Höhe erreichen, die wenigstens dem Abstand zwischen dem Röntgendetektor 4 und der Kompressionseinheit 9 im vollständig komprimierten Endzustand entspricht. Mit anderen Worten kann die Brust 8, wenn sie sich in ihrer endgültigen Aufnahmeposition befindet, seitlich vollständig von Kammern 30, 31, ... des Luftkissens 18 umgeben und von deren Anlageflächen 19 beaufschlagt sein.

Im einfachsten Fall ist das Luftkissen 18 überall gleich dick bzw. das Luftkissen 18 ist über seine gesamte Länge mit identischen Kammern 30, 31, ... ausgestattet. Vorteilhafterweise sind jedoch, je nachdem, in welchem Bereich des Luftkissens 18 sich die Kammern 30, 31, ... befinden, unterschiedlich große und/oder unterschiedlich geformte Kammern 30, 31, ... vorgesehen. So können beispielsweise die im Bereich der Brustvorderseite, also in dem anzuhebenden vorderen Bereich 21 der Brust 8, unterhalb der Brust 8 angeordneten Kammern 30, 31, ... des Luftkissens 18 im aufgeblasenen, befüllten Zustand dicker (höher) sein als die Kammern, die sich in einem hinteren Bereich unterhalb der Brust 8 befinden, in dem die Brust 8 bereits beidseitig zwischen der Kompressionseinheit 9 und dem Röntgendetektor 4 komprimiert und fixiert ist.

Das Befüllen und Entleeren der Kammern 30, 31, ... erfolgt stets auf definierte, d.h. nicht willkürliche Art und Weise. Insbesondere kann die Reihenfolge und/oder die Geschwindigkeit des Befüllens und Entleerens der Kammern 30, 31, ... mit dem Vorgang der Kompression und Fixierung der Brust 8 mittels der Kompressionsanordnung 6 abgestimmt sowie an den jeweiligen Anwendungsfall angepasst sein. So müssen beispielsweise während der Positionierung der Brust 8 nicht sämtliche Kammern 30, 31, ... des Luftkissens 18 befüllt sein oder werden. Auch können Kammern 30, 31, ... während des Positioniervorgangs zunächst befüllt und anschließend, z.B. zeitgleich mit dem Befüllen einer benachbarten Kammer, wieder entleert werden, beispielsweise um eine bestimmte Formgebung der Brust 8 zu erreichen. Auch kann eine Kammer 30, 31, ... während des Positionierens mehrfach befüllt und entleert werden oder das Befüllen (oder Entleeren) findet in mehreren zeitlich getrennten Schritten statt. Bei Beginn des Positioniervorgangs können Kammern 30, 31, ... bereits teilweise oder vollständig befüllt sein, beispielsweise um eine bevorzugte Ausgangsposition der Brust 8 für den nachfolgenden Positioniervorgang bereitzustellen.

Während des Befüllens werden zunächst diejenigen Bereiche des Luftkissens 18 befüllt, die dem Befüllungsvorgang wenig Widerstand entgegenbringen. Dies sind typischerweise Bereiche, in denen sich die Kammern 30, 31, ... befüllen lassen, ohne dass sie sich gegen die Brust 8 legen. Auf diese Weise füllen sich zunächst alle nicht unmittelbar zur Beaufschlagung beitragenden Kammern 30, 31, .... Bei einer weiteren Erhöhung des Luftdrucks erfolgt dann die gewünschte Beaufschlagung der Brust 8 in den ausgewählten Bereichen.

Die Füllgeschwindigkeit kann in Abhängigkeit von dem Befüllungsgrad der jeweiligen Kammer 30, 31, ... geregelt werden, insbesondere derart, dass die Füllgeschwindigkeit sinkt, je näher die Kammer 30, 31, ... ihrem maximalen Befüllungsgrad oder ihrem üblichen Befüllungsgrad kommt, und/oder in Abhängigkeit von dem eingestellten Abstand zwischen dem Röntgendetektor 4 und der Kompressionseinheit 9. Auf diese Weise kann die für das Befüllen erforderliche Zeit minimiert werden. Zum Entleeren der Kammern 30, 31, ... kann die eingefüllte Luft einfach wieder auf den Kammern 30, 31, ... entlassen werden, beispielsweise durch ein Auslassventil oder dergleichen. Bei zeitkritischen Anwendungen kann ein schnelleres Entleeren der Kammern 30, 31, ... auch durch Auspumpen erfolgen.

Im einfachsten Fall ist das Luftkissen 18 zu Beginn des Positioniervorgangs nicht aufgeblasen. Anschließend wird die Kompressionseinheit 9 abgesenkt, bis die Gaze 11 leicht auf die Oberseite 29 der Brust 8 drückt und diese vorfixiert. Jetzt wird das Luftkissen 18 entsprechend der gewünschten Zielposition der Brust 8 befüllt, die Brust 8 wird von dem Luftkissen 18 eingefasst und gegen die Gaze 11 gedrückt.

Zum Befüllen und Entleeren der Kammern 30, 31, ... des Luftkissens 18 dient ein an das Luftkissen 18, genauer gesagt die Anzahl der Kammern 30, 31, ... anzuschließendes pneumatisches System, wie in FIG 2 angedeutet, mit wenigstens einer vorzugsweise (elektro)motorisch antreibbaren Pumpe 25 sowie weiteren Elementen, wie beispielsweise einer Anzahl von Ventilen, Verteilerstücken, Verbindungs- bzw. Anschlussleitungen 26 usw. Sind einzeln bzw. individuell befüllbare Kammern 30, 31, ... vorgesehen, dann verfügen diese über eine Absperrfunktion, so dass das Befüllen ausgewählter Kammern 30, 31, ... wahlweise unterdrückt oder freigegeben werden kann. Aufbau und Funktion eines solchen Systems sind dem Fachmann bekannt und brauchen daher an dieser Stelle nicht näher ausgeführt werden. Sämtliche aus nichtröntgendurchlässigem oder allgemein für die Bildaufnahmen störendem Material gefertigten Elemente sind dabei vorzugweise außerhalb des Untersuchungsbereiches angeordnet, um Bildartefakte und dergleichen während der Bildgebung zu vermeiden.

Im einfachsten Fall ist das pneumatische System manuell bedienbar. So können die Pumpe 25 und die Bedienelemente, wie Absperr- bzw. Durchlasselemente für die Anschlussleitungen 26, von Hand betätigt werden.

In einer anderen Variante wird die Bedienperson durch eine visuelle Anzeige der Lage und Form der Brust 8 unterstützt. Die Betätigung der Bedienelemente erfolgt nach wie vor manuell. Die Anzeige stellt die aktuelle Brustpositionierung dar, vorzugsweise durch ein Bild der Brust 8 von oben (Draufsicht) und/oder von vorn. Als Anzeigegerät dient beispielsweise der Bildschirm 14.

In einer weiteren Ausführung ist eine ferngesteuerte Betätigung der Bedienelemente des pneumatischen Systems vorgesehen. In diesem Fall ist ebenfalls die visuelle Anzeige der Brustposition vorgesehen. Dabei kann erneut der Bildschirm 14 als Anzeigegerät und eine separate Eingabeeinheit 15 verwendet werden. Alternativ dient das Anzeigegerät zugleich als Eingabeeinheit zur manuellen Steuerung, zu welchem Zweck ein Touchscreen oder dergleichen als Benutzerschnittstelle verwendet werden kann (nicht dargestellt).

In einer alternativen Ausführung ist eine halb- oder vollautomatische Betätigung der Bedienelemente anhand ermittelter Daten vorgesehen. Während im halbautomatischen Modus die visuelle Anzeige verwendet wird, um mittels einer Eingabefunktion einzelne Bedienaktionen durchzuführen oder zu bestätigen, ist im vollautomatischen Modus, abgesehen gegebenenfalls von einer Bestätigungs- oder Freigabefunktion, eine Interaktion mit der Bedienperson nicht mehr vorgesehen.

Es ist dann vorgesehen, die Brustpositionierung in Abhängigkeit von der Größe und Lage der Brust 8 selbständig durch ein automatisches Befüllen und Entleeren vornehmen zu lassen, dies in Abstimmung mit dem üblichen Kompressions- und Fixierungsvorgang mittels der Kompressionsanordnung 6, hier dem Absenken der Kompressionseinheit 9.

Bei den ermittelten Daten handelt es sich vorzugsweise um brustbezogene Daten, insbesondere um Daten zur Größe, Lage und/oder Position der Brust 8. Bei den ermittelten Daten handelt es sich darüber hinaus vorzugsweise um luftkissenbezogene Daten, insbesondere um Daten zum Füllstatus der Kammern 30, 31, ... bzw. zum Kammerdruck. Das Befüllen und/oder Entleeren der Kammern 30, 31, ... bzw. eine Änderung des Kammerdrucks erfolgt automatisch mit Hilfe einer Steuereinheit zur Steuerung des Befüllungs-/Entleerungsmechanismus, wobei diese Steuereinheit Steuerbefehle verarbeitet, die unter Verwendung der ermittelten Daten erstellt werden. Bei dieser Steuereinheit handelt es sich vorzugweise um die Steuer- und Recheneinheit 13 des Untersuchungsgerätes 1, welche diese Funktion ebenfalls realisiert. Dabei wird ein geeigneter Algorithmus verwendet, der auf der Basis der ermittelten Daten und des eingestellten Aufnahmetyps (z.B. Mammographie, Tomosynthese, kombinierte Aufnahme) die optimale Kompression und/oder Fixierung und/oder Positionierung der Brust 8 berechnet und diese, gegebenenfalls nach Bestätigung durch die Bedienperson, ausführt, kontrolliert und gegebenenfalls korrigiert.

Der Druck innerhalb der Kammern 30, 31, ... kann mittels Drucksensoren (nicht dargestellt) erfasst werden, wobei diese zur Vermeidung von Artefakten vorzugsweise außerhalb des Luftkissens 18 angeordnet sind. Länge, Breite und Höhe bzw. die Lage und/oder Position der Brust 8 können mit Hilfe von Lage- und/oder Abstandssensoren (nicht dargestellt) oder mit Hilfe einer in der Nähe des Untersuchungsgerätes platzierten Foto- oder Videokamera (nicht dargestellt) erfasst werden, wobei die Foto- bzw. Videodaten bzw. alle erfassten Daten zum Zweck der weiteren Verwendung in der Steuer- und Recheneinheit 13 aufbereitet bzw. weiterverarbeitet werden. Darüber hinaus können auch andere geeignete Parameter durch die Sensorik erfasst und über die Steuereinheit 13 zur Steuerung des pneumatischen Systems, insbesondere zur Steuerung der Pumpe 25 und der Bedienelemente, also z.B. der Absperrelemente, verwendet werden.

Um die Bildgebung nicht zu beeinflussen, insbesondere um das Auftreten von Streustrahlung und Bildartefakten zu vermeiden, ist das Luftkissen 18 vorzugsweise aus einem für Röntgenstrahlen durchlässigen Material hergestellt, insbesondere aus einem biegsamen Kunststoffmaterial, und die Kammerwände weisen eine vergleichsweise geringe Materialstärke auf. Auch im befüllten Zustand ist das Luftkissen 18 bzw. sind dessen Kammern 30, 31, ... zu einem bestimmten Maß flexibel, so dass diese sich an die Form der mit dem gewünschten Anpressdruck zu beaufschlagenden Brust 8 anpassen können. Hierdurch wird eine besonders schonende Handhabung der Brust 8 während der Positionierung, insbesondere der Formung und/oder der Bewegung, erreicht.

Zusätzlich zu einer unterschiedlichen Befüllung einzelner Kammern 30, 31, ... des Luftkissens 18, d.h. einer Befüllung mit unterschiedlichem Druck, oder auch anstelle einer druckverschiedenen Befüllung, kann die Form- und Volumengebung des Luftkissens 18 in einer Ausführungsform der Erfindung dadurch gezielt beeinflusst werden, dass zumindest für bestimmte Abschnitte oder Teile des Luftkissens 18 oder seiner Kammern 30, 31, ... unterschiedliche Materialstärken der Kammerwände verwendet werden. Mit anderen Worten sind erste Bereiche, die bei gleichem Fülldruck ein geringes Volumen aufweisen sollen als zweite Bereiche, mit dickeren Kammerwänden ausgestattet bzw. zweite Bereiche, die ein größeres Volumen aufweisen sollen, sind mit dünneren Kammerwänden versehen. Damit kann, ohne eine Unterteilung in druckverschiedene Bereiche, eine bestimmte gewünschte Formgebung einer Kammer 30, 31, ... bei gleichem Innendruck erreicht werden. Hierdurch lässt sich die Anzahl der benötigten Kammern 30, 31, ... und damit die Anzahl der erforderlichen pneumatischen Anschlüsse etc. verringern.

Vorzugsweise ist das Luftkissen 18 für den Einmalgebrauch ausgeführt, d.h. es braucht nach den Aufnahmen nicht gereinigt werden. In dem dargestellten Ausführungsbeispiel ist das Luftkissen 18 an dem Röntgendetektor 4 befestigt. Damit das Luftkissen 18 schnell getauscht werden kann, ist es vorzugsweise mit Schnellverbindern zur Ausbildung einer Rast-, Schnapp- oder Klemmverbindung oder mit einem Klettverschluss versehen (nicht abgebildet), so dass ein Lösen und Befestigen des Luftkissens 18 mit wenigen Handgriffen möglich ist. Vorzugsweise ist das Luftkissen 18 an seiner Außenseite antibakteriell beschichtet oder auf andere Weise mit antibakteriellen Eigenschaften ausgestattet.

Überdeckt das Luftkissen 18 die von der Brust 8 belegbare Auflagefläche des Röntgendetektors 4 im Wesentlichen vollständig, dann verhindert es eine Benetzung und damit Verschmutzung des Röntgendetektors 4 mit Ultraschall-Gel. Das Reinigen des Röntgendetektors 4 kann daher entfallen. Gleichzeitig gewährleistet die Verwendung eines den Röntgendetektor 4 im Wesentlichen vollständig bedeckenden Luftkissens 18, dass Brüste 8 unabhängig von ihrer Größe nach dem Platzieren auf dem Röntgendetektor 4 von dem Luftkissen 18 positioniert werden können.

Einige Kammern 30, 31, ... des Luftkissens 18 sind vorzugsweise derart angeordnet, dass sie im befüllten Zustand von der Unterseite 27 der Brust 8 kommend im Bereich der Brustwarze 22 nach oben geführt sind. Dadurch wird sichergestellt, dass die brustwandnahen Bereiche der Brust 8, insbesondere im Bereich der Brustwarze 22, mit dem Positionierelement 18 angehoben werden, also diejenigen Abschnitte der Brust 8, deren Gewebedicke so gering ist, dass sie nach der üblichen Kompression und Fixierung mittels der Kompressionseinheit 9 nicht an der Kompressionsfläche 16 anliegen und daher nicht zu einem Ultraschallbild beitragen können. Typischerweise wird mit anderen Worten die Brustvorderseite bzw. der vordere Brustbereich 21 angehoben.

Nach der Kompression und Fixierung der Brust 8 mittels der Kompressionseinheit 9 und im Anschluss an das oben beschriebene Positionieren der Brust 8, werden die benötigten Röntgenbilder der Brust 8 aufgenommen. Anschließend erfolgen die Ultraschallaufnahmen. Hierdurch wird eine besonders gute Ankopplung der Brust 8 an den Ultraschallkopf 5 und damit eine besonders gute Abdeckung der Brustfläche von bis zu 100 Prozent erreicht. Die während der Aufnahme der Röntgenbilder vorliegende Kompression und Fixierung der Brust 8, einschließlich der zusätzlichen Positionierung durch das Luftkissen 18, bleibt während der Aufnahme der Ultraschallbilder aufrechterhalten. Aufgrund der gleichbleibenden Positionierung der Brust 8 während des gesamten Untersuchungszeitraums können die Bildinhalte beider Modalitäten einfacher zueinander in Beziehung gesetzt werden, was eine lokale Zuordnung verdächtiger Regionen vereinfacht. Dies führt zu einer verbesserten Diagnostik.

Mit dem Einsatz der Erfindung kann auch ohne Verwendung unterschiedlich großer Kompressionseinheiten 9 auf einfache Art und Weise eine patientenindividuelle und gezielte Brustpositionierung erfolgen. Durch eine fallweise optimierte Brustpositionierung können spezielle klinische Fragestellungen sowie typische Brustformen individuell berücksichtigt werden. Dabei können nicht nur die hier näher beschriebenen erfindungsgemäßen Kompressionseinheiten 9 mit dem Untersuchungsgerät verbunden werden. Auch herkömmliche Kompressionseinheiten 9 mit flachen, starren Kompressionsflächen, beispielsweise aus Plexiglas, können fallweise zum Einsatz kommen, beispielsweise dann, wenn lediglich eine Röntgenuntersuchung (Mammographie und/oder Tomosynthese) der Brust 8, nicht aber eine Ultraschalluntersuchung erfolgen soll. In diesem Fall kann das Positionierelement, hier das Luftkissen 18, ausschließlich oder zusätzlich als Kompressionseinheit 9 dienen und die Brust 8 auf den Röntgendetektor 4 drücken. Auch kann in diesem Fall, also wenn ausschließlich Röntgenaufnahmen erfolgen sollen, das Luftkissen 18 so ausgeführt sein, dass Kammern oberhalb der Brust 8 vorgesehen sind. Diese oberen Kammern (nicht abgebildet) können zur Ausbildung einer brustindividuell geformten Kompressionsfläche 16 dienen und formen die Brust 8 von oben. Diese oberen Kammern können allein oder in Kombination mit unteren und/oder seitlichen Kammern 30, 31, ... eingesetzt werden.

In allen beschriebenen Fällen können anstelle eines einzigen Luftkissens 18 mehrere Luftkissen 18 zum Einsatz kommen. Diese sind dann vorzugsweise miteinander mechanisch verbunden, so dass sich ein Positionierelementeverbund ergibt, der ähnlich wie die einzelnen Kammern 30, 31, ... eines einzigen Positionierelements betrieben werden kann.

Obwohl die Erfindung im Detail durch das bevorzugte Ausführungsbeispiel näher illustriert und beschrieben wurde, so ist die Erfindung nicht auf die offenbarten Beispiele eingeschränkt und andere Variationen können vom Fachmann hieraus abgeleitet werden, ohne den Schutzumfang der Erfindung zu verlassen. Insbesondere gelten die im Zusammenhang mit der Verwendung von Kompressionsmulden gemachten Angaben stets auch für andere Arten von Kompressionseinheiten.

### Bezugszeichenliste

- 1: Untersuchungsgerät
- 2: Trajektorie
- 3: Röntgenstrahler
- 4: Röntgendetektor, Detektor
- 5: Ultraschalleinheit, Ultraschallkopf
- 6: Kompressionsanordnung
- 7: Untersuchungsbereich
- 8: Objekt, Brust
- 9: obere Kompressionseinheit, Kompressionsmulde
- 10: untere Kompressionseinheit, Detektoroberseite
- 11: Kompressionselement, Gewebe, Gaze
- 12: Rahmen
- 13: Steuer- und Recheneinheit
- 14: Bildschirm
- 15: Eingabeeinheit
- 16: Kompressionsfläche
- 17: Trägerelement, Stativ
- 18: Positionierelement
- 19: Anlagefläche
- 20: mittlerer Brustbereich
- 21: vorderer Brustbereich
- 22: Brustwarze
- 23: rechter Seitenabschnitt
- 24: linker Seitenabschnitt
- 25: Pumpe
- 26: Verbindungs- oder Anschlussleitung
- 27: Brustunterseite
- 28: Brustlängsrichtung
- 29: Brustoberseite
- 30: untere Kammer (rechts)
- 31: untere Kammer (links)
- 32: seitliche Kammer (rechts)
- 33: seitliche Kammer (links)

## Patentansprüche

1. Röntgen-Untersuchungsgerät (1), ausgeführt als kombiniertes Röntgen-/Ultraschall-Untersuchungsgerät (1), mit einem Röntgenstrahler (3) und einem Röntgendetektor (4) zur Aufnahme wenigstens eines Röntgenbildes einer Brust (8), mit einer Ultraschalleinheit (5) zur Aufnahme wenigstens eines Ultraschallbildes der Brust (8), mit einer Kompressionseinheit (9) zur Kompression und Fixierung der Brust (8), wobei die Kompressionseinheit (9) ein für Röntgenstrahlen und für Ultraschall durchlässiges Kompressionselement (11) aufweist, und wobei die Ultraschalleinheit (5) entlang des Kompressionselements (11) über die Brust (8) führbar ist, **gekennzeichnet durch** ein volumen- und/oder formvariables Positionierelement (18) zur Beaufschlagung der Brust (8) derart, dass das Positionierelement (18) die Brust (8) verformt und sich die Brust (8) an das Kompressionselement (11) anlegt, wobei das Positionierelement (18) als ein befüllbares und entleerbares Kissen ausgeführt ist, das mindestens eine Kammer (30, 31, ...) aufweist und wobei die Volumen- und/oder Formvariabilität des Positionierelements (18) durch ein Befüllen mindestens einer Kammer (30, 31, ...) des Positionierelements (18) mit einem Füllmedium und/oder durch ein Entleeren mindestens einer Kammer (30, 31, ...) des Positionierelements (18) bewirkbar ist.

2. Röntgen-Untersuchungsgerät (1) nach Anspruch 1, **dadurch gekennzeichnet, dass** das Positionierelement (18) ein aufblasbares Luftkissen ist.

3. Röntgen-Untersuchungsgerät (1) nach Anspruch 1 oder 2, **dadurch gekennzeichnet, dass** die Volumen- und/oder Formvariabilität des Positionierelements (18) durch die Materialstärke der Wände der Kammer (30, 31, ...) beeinflusst ist.

4. Röntgen-Untersuchungsgerät (1) nach einem der Ansprüche 1 bis 3, **dadurch gekennzeichnet, dass** das Positionierelement (18) mehrere Kammern (30, 31, ...) aufweist, die unabhängig voneinander befüllbar und entleerbar sind.

5. Verfahren zur Positionierung einer Brust (8) in einem als kombiniertes Röntgen-/Ultraschall-Untersuchungsgerät (1) ausgeführten Röntgen-Untersuchungsgerät (1) mit einem Röntgenstrahler (3) und einem Röntgendetektor (4) zur Aufnahme wenigstens eines Röntgenbildes der Brust (8), mit einer Ultraschalleinheit (5) zur Aufnahme wenigstens eines Ultraschallbildes der Brust (8), mit einer Kompressionseinheit (9) zur Kompression und Fixierung der Brust (8), wobei die Kompressionseinheit (9) ein für Röntgenstrahlen und für Ultraschall durchlässiges Kompressionselement (11) aufweist, und wobei die Ultraschalleinheit (5) entlang des Kompressionselements (11) über die Brust (8) führbar ist, **dadurch gekennzeichnet, dass** eine Positionierung der Brust (8) unter Verwendung eines zur Beaufschlagung der Brust (8) ausgebildeten, volumen- und/oder formvariablen Positionierelements (18) erfolgt derart, dass das Positionierelement (18) die Brust (8) verformt und sich die Brust (8) an das Kompressionselement (11) anlegt, wobei das Positionierelement (18) als ein befüllbares und entleerbares Kissen ausgeführt ist, das mindestens eine Kammer (30, 31, ...) aufweist und wobei die Volumen- und/oder Formvariabilität des Positionierelements (18) durch ein Befüllen mindestens einer Kammer (30, 31, ...) des Positionierelements (18) mit einem Füllmedium und/oder durch ein Entleeren mindestens einer Kammer (30, 31, ...) des Positionierelements (18) bewirkt wird.

6. Verfahren nach Anspruch 5, **dadurch gekennzeichnet, dass** die Beaufschlagung der Brust (8) durch das Positionierelement (18) mittels der Kompressionseinheit (9) vor, während und/oder nach der Kompression und Fixierung der Brust (8) erfolgt derart, dass sich die Brust (8) an das Kompressionselement (11) anlegt, bevor das wenigstens eine Röntgenbild der Brust (8) aufgenommen wird.

7. Verfahren nach Anspruch 6, **dadurch gekennzeichnet, dass** die während der Aufnahme des wenigstens eines Röntgenbildes vorliegende Kompression und Fixierung der Brust (8) während der Aufnahme des wenigstens eines Ultraschallbildes aufrechterhalten bleibt.

8. Verfahren nach Anspruch 5, **dadurch gekennzeichnet, dass** das Positionierelement (18) als Kompressionseinheit (9) dient.

9. Verfahren nach einem der Ansprüche 5 bis 8, **dadurch gekennzeichnet, dass** die Kammern (30, 31, ...) des Positionierelements (18) in einer definierten Reihenfolge befüllt und/oder entleert werden.

10. Verfahren nach einem der Ansprüche 5 bis 9, **dadurch gekennzeichnet, dass** der Druck innerhalb der Kammern (30, 31, ...) definiert verändert wird.

11. Verfahren nach einem der Ansprüche 5 bis 10, **dadurch gekennzeichnet, dass** brustbezogene und/oder positionierelementbezogene Daten ermittelt werden und das Befüllen und/oder Entleeren der Kammern (30, 31, ...) entsprechend dieser Daten erfolgt.

12. Verfahren nach einem der Ansprüche 5 bis 11, **gekennzeichnet durch** ein visuelles Anzeigen der Position der Brust (8) durch ein Anzeigegerät (14), wobei die Anzeige eine Bedienperson bei dem Betätigen von Bedienelementen eines pneumatischen Systems zum Befüllen und Entleeren der Kammern (30, 31, ...) des Positionierelements (18) unterstützt.

13. Verfahren nach einem der Ansprüche 5 bis 11, **gekennzeichnet durch** ein Ermitteln von brustbezogenen Daten, insbesondere von Größe, Lage und/oder Position der Brust (8), und ein Ermitteln von Daten zum Füllstatus der Kammern (30, 31, ...) oder zum Kammerdruck sowie ein automatisches Befüllen und Entleeren der Kammern (30, 31, ...) des Positionierelements (18) unter Verwendung der ermittelten Daten.

## Claims

1. X-ray examination device (1) embodied as a combined X-ray/ultrasound examination device (1), with an X-ray tube (3) and an X-ray detector (4) for recording at least one X-ray image of a breast (8), with an ultrasound unit (5) for recording at least one ultrasound image of the breast (8), with a compression unit (9) for compression and fixation of the breast (8), wherein the compression unit (9) comprises a compression element (11) penetrable by X-rays and ultrasound, and wherein the ultrasound unit (5) can be guided along the compression element (11) over the breast (8), **characterised by** a volume-variable and/or shape-variable positioning element (18) for acting on the breast (8) such that the positioning element (18) deforms the breast (8) and the breast (8) rests on the compression element (11), wherein the positioning element (18) is embodied as a fillable and emptiable cushion comprising at least one chamber (30, 31, ...) and wherein the volume-variability and/or shape-variability of the positioning element (18) can be effected by filling at least one chamber (30, 31, ...) of the positioning element (18) with a filling medium and/or by emptying at least one chamber (30, 31, ...) of the positioning element (18).

2. X-ray examination device (1) according to claim 1, **characterised in that** the positioning element (18) is an inflatable air cushion.

3. X-ray examination device (1) according to claim 1 or 2, **characterised in that** the volume-variability and/or shape-variability of the positioning element (18) is influenced by the material thickness of the walls of the chamber (30, 31, ...).

4. X-ray examination device (1) according to one of claims 1 to 3, **characterised in that** the positioning element (18) comprises a plurality of chambers (30, 31, ...) that can be filled and emptied independently of one another.

5. Method for positioning a breast (8) in an X-ray examination device (1) embodied as a combined X-ray/ultrasound examination device (1) with an X-ray tube (3) and an X-ray detector (4) for recording at least one X-ray image of the breast (8), with an ultrasound unit (5) for recording at least one ultrasound image of the breast (8), with a compression unit (9) for compression and fixation of the breast (8), wherein the compression unit (9) comprises a compression element (11) penetrable by X-rays and ultrasound and wherein the ultrasound unit (5) can be guided along the compression element (11) over the breast (8), **characterised in that** the breast (8) is positioned using a volume-variable and/or shape-variable positioning element (18) for acting on the breast (8) such that the positioning element (18) deforms the breast (8) and the breast (8) rests on the compression element (11), wherein the positioning element (18) is embodied as a fillable and emptiable cushion comprising least one chamber (30, 31, ...) and wherein the volume-variability and/or shape-variability of the positioning element (18) is effected by filling at least one chamber (30, 31, ...) of the positioning element (18) with a filling medium and/or by emptying at least one chamber (30, 31, ...) of the positioning element (18).

6. Method according to claim 5, **characterised in that** the action of the positioning element (18) on the breast (8) by means of the compression unit (9) is performed before, during and/or after compression and fixation of the breast (8) such that the breast (8) rests on the compression element (11) before the at least one X-ray image of the breast (8) is recorded.

7. Method according to claim 6, **characterised in that** the compression and fixation of the breast (8) provided during the recording of the at least one X-ray image is maintained during the recording of the at least one ultrasound image.

8. Method according to claim 5, **characterised in that** the positioning element (18) is used as the compression unit (9).

9. Method according to one of claim 5 to 8, **characterised in that** the chambers (30, 31, ...) of the positioning element (18) are filled and/or emptied in a defined series.

10. Method according to one of claims 5 to 9, **characterised in that** the pressure inside the chambers (30, 31, ...) is changed in a defined manner.

11. Method according to one of claims 5 to 10, **characterised in that** breast-related and/or positioning-element-related data is ascertained and the filling and/emptying of the chambers (30, 31, ...) is performed in accordance with this data.

12. Method according to one of claims 5 to 11, **characterised by** a visual display of the position of the breast (8) by a display device (14), wherein the display supports an operator in the actuation of operating elements of a pneumatic system for filling and emptying the chambers (30, 31, ...) of the positioning element (18).

13. Method according to one of claims 5 to 11, **characterised by** the ascertainment of breast-related data, in particular the size, location and/or position of the breast (8), and the ascertainment of data on the filling status of the chambers (30, 31, ...) or on the chamber pressure and automatic filling and emptying of the chambers (30, 31, ...) of the positioning element (18) using the ascertained data.

## Revendications

1. Appareil (1) d'examen radiologique réalisé en appareil (1) d'examen combiné à rayons X/ultrasons, comprenant un émetteur (3) de rayons X et un détecteur (4) de rayons X pour l'enregistrement d'au moins une image par rayons X d'un sein (8), comprenant une unité (5) d'ultrasons pour l'enregistrement d'au moins une image par ultrasons du sein (8), comprenant une unité (9) de compression pour comprimer et immobiliser le sein (8), l'unité (9) de compression ayant un élément (11) de compression perméable au rayonnement X et aux ultrasons, et dans lequel l'unité (5) d'ultrasons peut être guidée sur le sein (8), le long de l'élément (11) de compression, **caractérisé par** un élément (18) de mise en position, de volume et/ou de forme variable pour s'appliquer au sein (8) de manière à ce que l'élément (18) de mise en position déforme le sein (8) et de manière à ce que le sein (8) s'applique à l'élément (11) de compression, l'élément (18) de mise en position étant réalisé sous la forme d'un coussin pouvant être rempli et vidé, qui a au moins une chambre (30, 31, ...) et dans lequel la variation de volume et/ou de forme de l'élément (18) de mise en position peut être provoquée en remplissant au moins une chambre (30, 31, ...) de l'élément (18) de mise en position d'un fluide de remplissage et/ou en vidant au moins une chambre (30, 31, ...) de l'élément (18) de mise en position.

2. Appareil (1) d'examen radiologique suivant la revendication 1, **caractérisé en ce que** l'élément (18) de mise en position est un coussin d'air gonflable.

3. Appareil (1) d'examen radiologique suivant la revendication 1 ou 2, **caractérisé en ce que** la variation de volume et/ou de forme de l'élément (18) de mise en position est influencée par l'épaisseur des parois des chambres (30, 31, ...).

4. Appareil (1) d'examen radiologique suivant l'une des revendications 1 à 3, **caractérisé en ce que** l'élément (18) de mise en position a plusieurs chambres (30, 31, ...), qui peuvent être remplies et vidées indépendamment les unes des autres.

5. Procédé de mise en position d'un sein (8) dans un appareil (1) d'examen radiologique réalisé en appareil (1) d'examen combiné à rayons X/ultrasons, comprenant un émetteur (3) de rayons X et un détecteur (4) de rayons X pour l'enregistrement d'au moins une image par rayons X d'un sein (8), comprenant une unité (5) d'ultrasons pour l'enregistrement d'au moins une image par ultrasons du sein (8), comprenant une unité (9) de compression pour comprimer et immobiliser le sein (8), l'unité (9) de compression ayant un élément (11) de compression perméable au rayonnement X et aux ultrasons, et dans lequel l'unité (5) d'ultrasons peut être guidée sur le sein (8), le long de l'élément (11) de compression, **caractérisé en ce qu'**une mise en position du sein (8) s'effectue en utilisant un élément (18) de mise en position constitué pour s'appliquer au sein (8) et de volume et/ou de forme variable, de manière à ce que l'élément (18) de mise en position déforme le sein (8) et que le sein (8) s'applique à l'élément (11) de compression, l'élément (18) de mise en position étant réalisé sous la forme d'un coussin pouvant être rempli et vidé, qui a au moins une chambre (30, 31, ...) et dans lequel on provoque la variation de volume et/ou de forme de l'élément (18) de mise en position en remplissant au moins une chambre (30, 31, ...) de l'élément (18) de mise en position d'un fluide de remplissage et/ou en vidant au moins une chambre (30, 31, ...) de l'élément (18) de mise en position.

6. Procédé suivant la revendication 5, **caractérisé en ce que** l'application de l'élément (18) de mise en position au sein (8), au moyen de l'unité (9) de compression, a lieu avant, pendant et/ou après la compression et l'immobilisation du sein (8), de manière à ce que le sein (8) s'applique à l'élément (11) de compression avant l'enregistrement de la au moins une image par rayons X du sein (8).

7. Procédé suivant la revendication 6, **caractérisé en ce que** la compression et l'immobilisation du sein (8) pendant l'enregistrement de la au moins une image à rayons X restent maintenues pendant l'enregistrement de la au moins une image à ultrasons.

8. Procédé suivant la revendication 5, **caractérisé en ce que** l'élément (18) de mise en position sert d'unité (9) de compression.

9. Procédé suivant l'une des revendications 5 à 8, **caractérisé en ce que** l'on remplit et/ou l'on vide les chambres (30, 31, ...) de l'élément (18) de mise en position dans un ordre défini.

10. Procédé suivant l'une des revendications 5 à 9, **caractérisé en ce que** l'on modifie, de manière définie, la pression dans les chambres (30, 31, ...).

11. Procédé suivant l'une des revendications 5 à 10, **caractérisé en ce que** l'on détermine des données se rapportant au sein et/ou à l'élément de position et l'on remplit et/ou vide les chambre (30, 31, ...) conformément à ces données.

12. Procédé suivant l'une des revendications 5 à 11, **caractérisé par** un affichage visuel de la position du sein (8) par un appareil (14) d'affichage, l'affichage assistant un opérateur dans le fonctionnement d'éléments de commande d'un système pneumatique pour remplir et vider les chambres (30, 31, ...) de l'élément (18) de mise en position.

13. Procédé suivant l'une des revendications 5 à 11, **caractérisé par** une détermination de données se rapportant au sein, notamment de la dimension, de l'emplacement et/ou de la position du sein (8) et une détermination de données sur l'état de remplissage des chambres (30, 31, ...) ou de la pression dans les chambres, ainsi qu'un remplissage et une vidange automatique des chambres (30, 31, ...) de l'élément (18) de mise en position, en utilisant les données déterminées.
